# EUROPEAN PATENT APPLICATION

(11) **EP 4 484 567 A1**
(43) Date of publication of application: **01.01.2025**
(21) Application number: 23756494.3
(22) Date of filing: 20.02.2023
(51) Int. Cl.: C12P 21/02, C12N 5/02, C12N 5/10, C12P 21/08

(54) **PERFUSION CULTURE METHOD**

(30) Priority: 21.02.2022 JP 2022024815
(71) Applicant: CHUGAI SEIYAKU KABUSHIKI KAISHA, Tokyo 115-8543 (JP); AJINOMOTO CO., INC., Chuo-ku Tokyo 104-8315 (JP)
(72) Inventor: TAKAO, Nobumasa, Tokyo 115-8543 (JP); HIGUCHI, Takuya, Kawasaki-shi, Kanagawa 210-8681 (JP); FUROMITSU, Shumpei, Kawasaki-shi, Kanagawa 210-8681 (JP)
(74) Representative: Vossius & Partner Patentanwälte Rechtsanwälte mbB
(86) International application number: PCT/JP2023/006038
(87) International publication number: WO 2023/157970

(57) **Abstract**

Provided are a perfusion culture method and the like that are able to improve the productivity in the production of recombinant protein. Specifically, a method for improving recombinant protein production in perfusion culture of an animal cell having a capacity to produce a recombinant protein, the method comprising adding cystine and/or ammonium iron citrate to a cell culture medium, and the like, are provided.

## Description

### [Technical Field]

The present invention relates to a perfusion culture method of animal cells. The present invention also relates to a method for producing a recombinant protein, a method for improving recombinant protein production, and an agent for improving recombinant protein production. The present invention further relates to a method for reducing a cell growth rate of animal cells.

### [Background Art]

In recent years, recombinant proteins such as therapeutic antibodies have become increasingly important in the pharmaceutical field. Most of the recombinant proteins approved as pharmaceuticals are produced by culturing animal cells that produce the recombinant proteins. However, the production of recombinant proteins requires higher costs than the production of low molecular compounds, and also the product requires higher quality.

Thus, the control and optimization of cell culture conditions aimed at improving the productivity and quality of recombinant proteins are crucial matters for the successful commercial production of recombinant proteins.

As one of the continuous methods of culture steps for improving the productivity of recombinant protein production by culturing animal cells, a perfusion culture method in which the culture fluid is continuously filtered and discharged from a culture tank, and a fresh medium containing nutrients is continuously supplied to the culture tank, has been used. According to this method, the cell density can be increased while constant nutrient and waste concentrations are maintained, thus the rate of protein production per volume of the culture tank can be improved. In addition, in the perfusion culture method, the produced recombinant proteins can be recovered immediately from the culture tank, thus high-quality recombinant proteins can be recovered, and even proteins that are unstable in the culture fluid can be efficiently recovered.

Various improvements have been attempted from various aspects to improve the productivity in perfusion culture, such as improvements of cultured cells, improvements of cell culture medium, and improvements of perfusion culture methods.

For example, International Publication No. WO2018/178069 describes a method of improving the productivity in perfusion culture by controlling the molar ratio of potassium ions to sodium ions in the cell culture medium.

### [Patent Literature]

International Publication No. WO2018/178069

### [Summary of Invention]

In perfusion culture, since the medium components and products are continuously extracted along with the waste products, the cost due to the loss of the medium and the purification cost due to the decrease in the concentration of the product can be increased.

An object of the present invention is to improve the productivity in the production of recombinant proteins.

That is, according to the present invention, the following aspects are provided.
[1] A method for improving recombinant protein production in perfusion culture of an animal cell having a capacity to produce a recombinant protein, the method comprising:
   adding cystine to a cell culture medium.
[2] A method for improving recombinant protein production per cell in perfusion culture of an animal cell having a capacity to produce a recombinant protein, the method comprising:
   adding cystine to a cell culture medium.
[3] The method according to [1] or [2], wherein the recombinant protein production is improved as compared to when cysteine of an equimolar concentration to the cystine in terms of cysteine is added.
[4] The method according to [3], wherein the recombinant protein production is improved by 10% or more.
[5] The method according to any one of [1] to [4], the method comprising adding cystine one hour or later after starting culturing.
[6] The method according to any one of [1] to [4], the method comprising adding cystine 6 hours or later after starting culturing.
[7] The method according to any one of [1] to [4], the method comprising adding cystine 24 hours or later after starting culturing.
[8] The method according to any one of [1] to [4], the method comprising adding cystine 72 hours or later after starting culturing.
[9] The method according to any one of [1] to [8], wherein a cysteine source concentration in a primary medium in the perfusion culture is 0.1 mM to 2.1 mM.
[10] A method for subjecting an animal cell having a capacity to produce a recombinant protein to perfusion culture, the method comprising:
   subjecting the animal cell having a capacity to produce a recombinant protein to perfusion culture in a cell culture primary medium having a cysteine source concentration of 0.1 mM to 2.1 mM; and
   adding cystine to a cell culture medium 72 hours or later after starting culturing.
[11] The method according to [10], wherein the capacity to produce a recombinant protein is improved as compared to when cysteine of an equimolar concentration to the cystine in terms of cysteine is added.
[12] The method according to [11], wherein the capacity to produce a recombinant protein is improved by 10% or more.
[13] A method for producing a recombinant protein, the method comprising:
   subjecting an animal cell having a capacity to produce a recombinant protein to perfusion culture in a cell culture primary medium having a cysteine source concentration of 0.1 mM to 2.1 mM;
   adding cystine to a cell culture medium 72 hours or later after starting culturing; and
   recovering a recombinant protein.
[14] The method according to [13], wherein the recombinant protein production is improved as compared to when cysteine of an equimolar concentration to the cystine in terms of cysteine is added.
[15] The method according to [14], wherein the recombinant protein production is improved by 10% or more.
[16] The method according to any one of [1] to [15], wherein a concentration of cystine after adding is 0.5 mM to 10 mM.
[17] The method according to any one of [1] to [15], wherein a concentration of cystine after adding is 1 mM to 10 mM.
[18] The method according to any one of [1] to [15], wherein a concentration of cystine after adding is 1.5 mM to 10 mM.
[19] The method according to any one of [1] to [15], wherein a concentration of cystine after adding is 2 mM to 10 mM.
[20] The method according to any one of [1] to [19], the method comprising adding a cystine-containing solution adjusted to pH 10 or higher.
[21] The method according to any one of [1] to [20], wherein the recombinant protein is an antibody.
[22] The method according to any one of [1] to [21], wherein the animal cell having a capacity to produce a recombinant protein contains a nucleic acid encoding a recombinant protein.
[23] The method according to any one of [1] to [22], wherein the animal cell is a mammalian cell.
[24] The method according to [23], wherein the mammalian cell is a CHO cell.
[25] The method according to [24], wherein the CHO cell is a CHO-K1 cell, a CHO-S cell, a CHO-DXB 11 cell, or a CHO-DG44 cell.
[26] The method according to any one of [1] to [25], wherein the cell culture medium in the perfusion culture contains ammonium iron citrate.
[27] An agent for improving recombinant protein production in perfusion culture of an animal cell having a capacity to produce a recombinant protein, the agent comprising cystine as an active ingredient.
[28] A method for improving recombinant protein production in perfusion culture of an animal cell having a capacity to produce a recombinant protein, the method comprising:
   subjecting the animal cell having a capacity to produce a recombinant protein to perfusion culture in a cell culture medium containing ammonium iron citrate.
[29] A method for improving recombinant protein production per cell in perfusion culture of an animal cell having a capacity to produce a recombinant protein, the method comprising:
   subjecting the animal cell having a capacity to produce a recombinant protein to perfusion culture in a cell culture medium containing ammonium iron citrate.
[30] The method according to [28] or [29], wherein the recombinant protein production is improved as compared to when the cell culture medium contains an alternative iron source to the ammonium iron citrate.
[31] The method according to [30], wherein the alternative iron source is sodium ferrous citrate.
[32] The method according to [30] or [31], wherein the recombinant protein production is improved by 10% or more.
[33] A method for subjecting an animal cell having a capacity to produce a recombinant protein to perfusion culture, the method comprising:
   subjecting the animal cell having a capacity to produce a recombinant protein to perfusion culture in a cell culture medium containing ammonium iron citrate.
[34] A method for reducing a cell growth rate in perfusion culture of an animal cell having a capacity to produce a recombinant protein, the method comprising:
   culturing the animal cell having a capacity to produce a recombinant protein in a cell culture medium containing ammonium iron citrate.
[35] The method according to [33] or [34], wherein the cell growth rate is reduced as compared to when the cell culture medium contains an alternative iron source to the ammonium iron citrate.
[36] The method according to [35], wherein the alternative iron source is sodium ferrous citrate.
[37] The method according to [35] or [36], wherein the cell growth rate is reduced by 10% or more.
[38] A method for reducing an amount of culture fluid discharged in perfusion culture of an animal cell having a capacity to produce a recombinant protein, the method comprising:
   subjecting the animal cell having a capacity to produce a recombinant protein to perfusion culture in a cell culture medium containing ammonium iron citrate,
   wherein the amount of culture fluid discharged is an amount of culture fluid discharged in a breeding step, and the breeding step is a step of discharging the culture fluid from a culture tank and adding a fresh medium in an equal amount to the discharged culture fluid to the culture tank.
[39] The method according to [38], wherein the amount of culture fluid discharged is reduced as compared to when the cell culture medium contains an alternative iron source to the ammonium iron citrate.
[40] The method according to [39], wherein the alternative iron source is sodium ferrous citrate.
[41] The method according to [39] or [40], wherein the amount of culture fluid discharged is reduced by 10% or more.
[42] A method for producing a recombinant protein, the method comprising:
   subjecting an animal cell having a capacity to produce a recombinant protein to perfusion culture in a cell culture medium containing ammonium iron citrate; and
   recovering a recombinant protein.
[43] The method according to any one of [28] to [42], wherein the cell culture medium contains an amount of ammonium iron citrate that provides an iron concentration of 30 mg/L to 200 mg/L.
[44] The method according to any one of [28] to [42], wherein the cell culture medium contains an amount of ammonium iron citrate that provides an iron concentration of 40 mg/L to 200 mg/L.
[45] The method according to any one of [28] to [42], wherein the cell culture medium contains an amount of ammonium iron citrate that provides an iron concentration of 50 mg/L to 150 mg/L.
[46] The method according to any one of [28] to [45], wherein the recombinant protein is an antibody.
[47] The method according to any one of [28] to [46], wherein the animal cell having a capacity to produce a recombinant protein contains a nucleic acid encoding a recombinant protein.
[48] The method according to any one of [28] to [47], wherein the animal cell is a mammalian cell.
[49] The method according to [48], wherein the mammalian cell is a CHO cell.
[50] The method according to [49], wherein the CHO cell is a CHO-K1 cell, a CHO-S cell, a CHO-DXB 11 cell, or a CHO-DG44 cell.
[51] The method according to any one of [28] to [50], the method comprising adding cystine to the cell culture medium.
[52] An agent for improving recombinant protein production in perfusion culture of an animal cell having a capacity to produce a recombinant protein, the agent comprising ammonium iron citrate as an active ingredient.
[53] A cell culture medium for perfusion culture of an animal cell having a capacity to produce a recombinant protein, the cell culture medium comprising cystine and ammonium iron citrate.

The present invention is able to improve the productivity in the production of recombinant proteins.

### [Brief Description of Drawings]

[Figure 1]

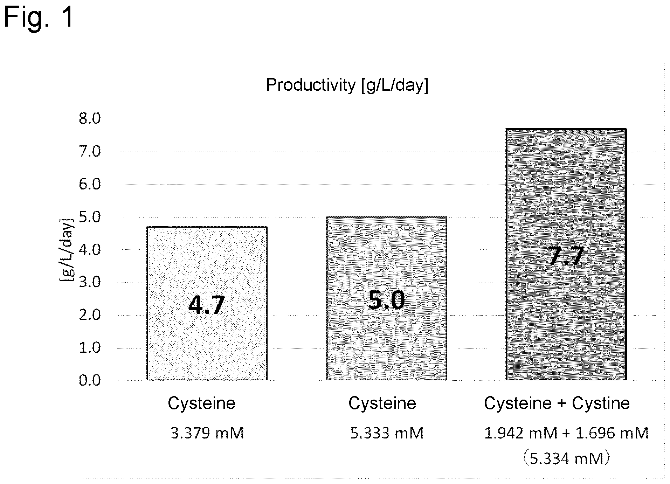

   Figure 1 is a graph showing differences in the productivity of recombinant proteins due to differences in cysteine source.
[Figure 2]

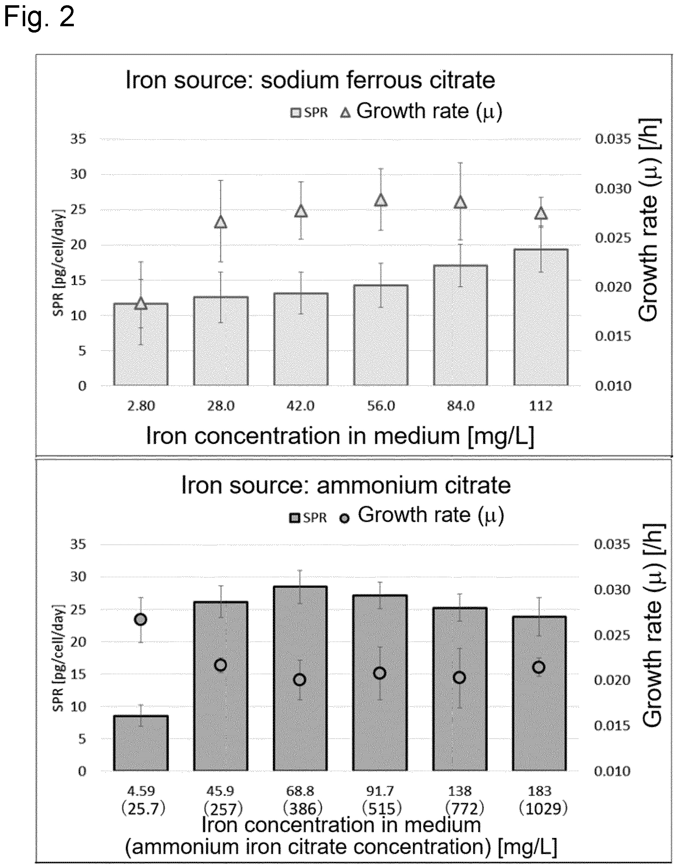

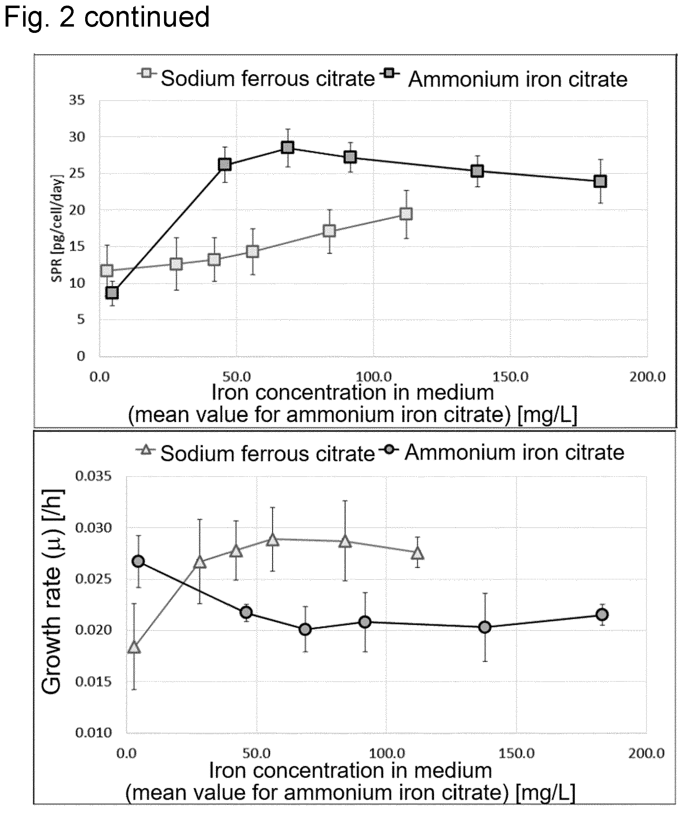

   Figure 2 is a graph showing differences in antibody production rate (SPR) and cell growth rate due to differences in iron source.

### [Description of Embodiments]

### (Description of terms)

In the present disclosure, unless otherwise noted, each term has the meaning set forth below.

The "recombinant protein" in the present disclosure refers to a polymer of amino acids of any length, produced using any gene recombinant technology. The polymer may be linear or branched, may comprise modified amino acids, and may comprise non-amino acids. The recombinant protein may also undergo chemical or biological modifications, such as disulfide bond formation, glycosylation, lipidation, acetylation, phosphorylation, amidation, derivatization by a protecting group or the like, cleavage, or binding to a labeling component (e.g., a radioisotope) or a drug (e.g., a toxin, a cytotoxic drug, or a radioisotope). Any molecule comprising a recombinant protein moiety as part of its structure is included in the recombinant protein of the present invention. The gene recombinant technology employed is not particularly limited as long as it is a technology that artificially engineers genes, but a typical example thereof is an introduction of a nucleic acid encoding a recombinant protein into a host cell.

Examples of the recombinant protein include, but are not limited to, an enzyme, a hormone, a cytokine, a clotting factor (or blood clotting-associated protein), an extracellular protein, a Notch ligand, an antibody, an antibody mimetic and an immunoadhesin produced using gene recombinant technology. These recombinant proteins are mainly used in the fields of pharmaceuticals, pesticides, foods, and other chemical industries, but are not limited to specific use.

Examples of the enzyme include alkaline phosphatase, β-lactamase, galactosidase, glucosidase, glucocerebrosidase, superoxide dismutase, and DNase.

Examples of the hormone include renin, growth hormone (e.g., human growth hormone or bovine growth hormone), parathyroid hormone, thyroid-stimulating hormone, calcitonin, gonadotropin (e.g., follicle-stimulating hormone, luteinizing hormone), glucagon, insulin (insulin A chain and insulin B chain), and proinsulin.

Examples of the cytokine include a tumor necrosis factor (e.g., TNF-α and -β), a vascular endothelial growth factor (VEGF), a brain-derived neurotrophic factor (BDNF), a neurotrophic factor (e.g., NT-3 to NT-6), a nerve growth factor (NGF), a platelet-derived growth factor (PDGF), a fibroblast growth factor (e.g., aFGF, bFGF), an epidermal growth factor (EGF), a transforming growth factor (e.g., TGF-α, TGF-β1 to TGF-β5), activin (e.g., activin A, activin C, activin E), an insulin-like growth factor (e.g., IGF-I, IGF-II), a hepatocyte growth factor (HGF), a keratinocyte growth factor, a stem cell factor (SCF), a bone morphogenetic protein (BMP), RANTES, erythropoietin (EPO), thrombopoietin (TPO), interferon-α, -β and -γ, a colony-stimulating factor (e.g., M-CSF, GM-CSF, G-CSF), and interleukin (e.g., IL-1 to IL-13).

Examples of the clotting factor and blood clotting-associated protein include Factor VII, Factor VIII, Factor VIIIC, Factor IX, Factor X, Factor XI, Factor XII, a tissue factor, a von Willebrand factor, protein C, protein S, plasminogen activator (e.g., urokinase, human urine or human tissue plasminogen activator (t-PA)), thrombin, prothrombin, thrombopoietin, thrombomodulin, antithrombin III, fibrinogen, and serum albumin.

Examples of the extracellular protein include fibronectin, vitronectin, collagen, osteopontin, laminin, and a partial sequence thereof.

Examples of the Notch ligand include DLL1, DLL3, DLL4, Jagged-1, and Jagged-2. The Notch ligand may be constructed, for example, as a fusion protein with an Fc region.

The "antibody" in the present disclosure refers to an immunoglobulin molecule having a binding ability to an antigen, and includes, but is not limited to, a full-length antibody having a polypeptide chain comprising a light chain and a heavy chain and a portion thereof (a fragment such as Fv, Fab, Fab', F(ab')₂, a VHH fragment, or the like). The heavy or light chain of each may comprise a variable region (related to recognition and binding to an antigen) and a constant region (related to localization and intercellular interaction). The most common full-length antibody includes two heavy chain constant regions (CH), two heavy chain variable regions (VH), two light chain constant regions (CL), and two light chain variable regions (VL). The variable region comprises a complementarity determining region (CDR) that is a sequence that confers antigen specificity to the antibody and a framework region (FR).

The antibody includes a monoclonal antibody, a multispecific antibody (e.g., a bispecific antibody) formed from two or more antibodies or antigen-binding sites, an antibody fragment having a desired biological activity, and a fusion protein comprising an antigen-binding site of an antibody. The antibody also includes a chimeric antibody (e.g., humanized antibody), a (fully) human antibody, a multivalent antibody, and a modified antibody.

The antibody may be of any class (e.g., IgG, IgA, IgM, IgD, IgE) and of any subclass (isotype).

The antibody may have undergone chemical or biological modifications, such as a conjugation to other molecules (such as, but not limited to, low or high molecular toxins (such as cytotoxic drugs) or radioisotopes), and these modified antibodies are also included in the antibody of the present invention.

The "antibody mimetic" in the present disclosure refers to a protein that is able to specifically bind to an antigen but not structurally related to an antibody. Specific examples of the antibody mimetic include a Z domain of protein A (affibody). More specifically, the antibody mimetic includes a ZHER2 affibody.

The recombinant protein is not limited to the above examples, and, for example, receptors of cytokines described above or the like, transferrin, various CD proteins, tumor-associated antigens (e.g., HER2, HER3 or HER4 receptors, or CA125), and viral antigens such as those for vaccines (e.g., viral envelope proteins) are also included in the recombinant protein. Fusion proteins containing any of the proteins described above are also included in the recombinant protein.

The "having a capacity to produce a recombinant protein " in the present disclosure means having a capacity to produce a protein using gene recombinant technology. The produced recombinant protein may be secreted extracellularly, i.e., into the culture medium, or may accumulate intracellularly, but is preferably secreted extracellularly.

Typically, a capacity to produce (express) a recombinant protein can be provided to a host cell by introducing a nucleic acid encoding the recombinant protein into the host cell. That is, a typical cell having a capacity to produce a recombinant protein is a cell containing a nucleic acid encoding a recombinant protein. Regarding the method for introducing a nucleic acid into a host cell, various methods are known in the art and any method known at the time of implementation can be used. Examples of such methods include a method of transforming a host cell with a vector containing a nucleic acid encoding a recombinant protein. The vector is preferably an expression vector containing a nucleic acid encoding a recombinant protein in an expressible form (e.g., in a form operably linked to a regulatory sequence such as a promoter, an enhancer, a ribosome binding sequence and/or a transcriptional termination sequence) and may be a plasmid.

Examples of the method of introducing a vector into a host cell include a method of physically or chemically introducing a vector into a host cell by an electroporation method, a particle gun method, a calcium phosphate method, or a lipofection method, or a method of infecting a host cell with a viral vector. The nucleic acid in the vector introduced into a host cell may be incorporated into the genome in the host cell by a genome editing technique such as CRISPR/Cas9. Alternatively, instead of introducing a nucleic acid encoding a recombinant protein into a host cell, a gene sequence on a genomic DNA can be directly modified by a genome editing technique such as CRISPR/Cas9 to encode a recombinant protein of interest, or a control sequence can be modified to control (e.g., improve) production of an endogenous protein of interest. All proteins produced in such a manner are also included in the recombinant protein of the present invention.

Any animal cell can be used as the "animal cell" in the present disclosure, but it is preferred that the animal cell is preferably an established cell line that is suitably used for producing a recombinant protein. Examples of the animal (animal species) include a mammal, a bird, an amphibian, and an insect. Preferred examples of the animal include an insect and a mammal, and particularly preferably, the animal is a mammal.

Examples of the insect cell include a cell of Spodoptera frugiperda. Examples of the cell of Spodoptera frugiperda include a Sf9 cell, a Sf21 cell, and a SF+ cell derived from the ovary of Spodoptera frugiperda.

Cells of primates such as human or monkey, and cells of rodents such as mouse, rat, or hamster, are preferably used as the mammalian cell.

Examples of the mammalian cell include, but are not limited to, cell lines such as HL-60 (ATCC No. CCL-240), HT-1080 (ATCC No. CCL-121), HeLa (ATCC No. CCL-2), 293 (ECACC No. 85120602), Hep G2 (ATCC No. HB8065); VERO (ATCC No. CCL-1651), CV1 (ATCC No. CCL70) and COS-7 (ATCC No. CRL-1651); NIH3T3 (ATCC No. CRL-1658), NS0 (ATCC No. CRL-1827); a Chinese hamster ovary cell (CHO cell), BHK21 (also referred to simply as a BHK cell; ATCC No. CRL-10), and MDCK (ATCC No. CCL-34) and sublines derived from these cell lines (e.g., a BHK TK-cell as sublines of BHK cell; and a CHO-K1 cell, a CHO-S cell, a CHO-DXB 11 cell (also referred to as CHO-DUKX cell or DuxB 11 cell), and a CHO-DG44 cell as sublines of CHO cell).

The "cell culture medium", "culture medium" and "medium" in the present disclosure refer to a solution containing a nutrient source used to proliferate or maintain cells, but when referring to the forms of selling or provision, it also includes stock solutions and powders (granules) for making the solution. The nutritional source includes essential ingredients and useful ingredients for cell growth and survival, and examples of such ingredients include carbohydrates such as sugar, lipids, nucleic acids, vitamins, essential and non-essential amino acids, essential elements, pH regulators, and solvents (water). The medium may be a serum-free medium or an animal protein-free medium.

The cell culture medium can be suitably selected from commercially available media and known media described in literatures, depending on the cells to be cultured and the purpose. The cell culture medium may be a combination of two or more media, the composition of the medium may be modified depending on the purpose of the culture and the cells, and animal serum, animal peptone and other animal-derived components, nutritional/growth factors, or hormones may be added to the medium. The cell culture medium is adjusted to a pH and salt concentration suitable for survival and growth of cells. The pH of the cell culture medium is, for example, 5.0 to 9.0, and the cell culture medium is often used in the range of preferably 6.0 to 8.0, more preferably 6.5 to 7.5.

Examples of the commercially available medium include RPMI-1640 (Roswell Park Memorial Institute 1640) medium, L-15 medium, MEM (Eagle's Minimum Essential Media), DMEM (Dulbecco's Modified Eagle Media), Iscoves' modified DMEM, Ham's F10 media, and F12 medium.

The "chemically defined cell culture medium" (CDM) in the present disclosure refers to a medium that does not contain animal-derived products such as animal serum or peptone, and in which all components are chemically defined. Like conventional cell culture media, the chemically defined cell culture medium can be suitably selected from commercially available media and known media described in literatures, depending on the cell lines to be cultured and the purpose. The cell culture medium may be a combination of two or more media, and animal serum and other animal components, nutritional/growth factors, or hormones may be added to the medium. The solution is adjusted to a pH and salt concentration optimal for survival and growth of cells.

Examples of the commercially available chemically defined cell culture media include AmpliCHO CD media, Dynamis(R) media, EX-CELL(R) Advanced(R) CHO fed-batch media, CD FortiCHO(R) media, CP OptiCHO(R), BalanCD(R) CHO Growth A media, ActiPro(R), MEM (Eagle's minimum essential media), DMEM (Dulbecco's Modified Eagle's media), RPMI-1640 (Roswell Park Memorial Institute 1640) media, CELLiST(R) Basal Media (BASAL3, BASAL10), and Feed Media (FEED2).

The "perfusion culture" in the present disclosure is a culture method in which cells are cultured while maintaining a medium amount at approximately constant by holding the cells in a culture tank (a reactor, also referred to as a culture vessel) during culture while continuously or intermittently discharging the cell culture medium from the culture tank and continuously or intermittently introducing a medium containing nutrients (fresh medium) into the culture tank (perfusion of the medium).

The holding of cells in a culture tank during culture requires separating the cells from the culture medium and retaining the cells in the culture tank. As the method for separating cells from the culture medium, filtration using a semi-permeable membrane or a filter (such as an alternating tangential flow perfusion filtration system), gravitational sedimentation, gravitational sedimentation, centrifugation, acoustic separation, or the like are used. In addition, many culture tanks, culture equipment, or culture systems having separation functions or the like suitable for perfusion culture are known and commercially available.

The cell culture medium used in perfusion culture can be the same as the cell culture medium used in other cell culture methods. In addition, the composition of the medium at the start of culturing (primary medium) and the composition of the medium used for subsequent perfusion (perfusion medium) may be the same or different (some components are increased or decreased, or another component is added).

The "cystine" as used in the present disclosure includes cystine (L-cystine), a cystine salt that functions as cystine after adding to the medium, and a hydrate thereof. However, when referring to a concentration of cystine, it means the concentration of cystine (L-cystine).

Examples of the cystine salt include cystine dihydrochloride, cystine disodium salt, and cystine disodium salt monohydrate. Examples of the hydrate include cystine disodium salt monohydrate. A plurality of cystine or cystine salts can be used in combination.

The "cysteine source" as used in the present disclosure refers to a compound that can be utilized as cysteine in a cell after a process such as degradation as required. Specific examples of the cysteine source include cysteine, cystine, and glutathione.

The "cysteine source concentration" as used in the present disclosure refers to a concentration of the cysteine source in terms of cysteine. For example, in the case of cystine, the cysteine source concentration is twice the cystine concentration, and in the case of glutathione, the cysteine source concentration is equal to the glutathione concentration.

The "ammonium iron citrate" used in the present disclosure is not particularly limited as long as it is ammonium iron(III) citrate consisting of a citrate ion, a ferric ion and an ammonium ion, or a hydrate thereof. For ammonium iron citrate, those of green color and reddish-brown color are known depending on the content of each ion, but both can be suitably used in embodiments of the present disclosure.

The "alternative iron source" as used in the present disclosure refers to an iron ion-containing molecule other than ammonium iron citrate that is available as an iron source for cell culture media. Examples of the alternative iron source include ferric phosphate, ferric pyrophosphate, ferric nitrate, ferrous sulfate, ferric chloride, ferrous lactate, ferric citrate, sodium ferrous citrate, sodium ferric ethylenediaminetetraacetate, dextran iron and hydrates thereof, and any combination thereof.

For the "iron concentration" (iron ion concentration) in the present disclosure, when the value of the iron content in the iron source (or information that can lead to the iron content such as the molecular formula of the iron source) can be obtained from the source (commercial source or the like), it is most preferable to use the value to calculate the theoretical value of the iron concentration, but when it is not possible to do so, the iron concentration (iron ion concentration) in the solution may be quantified using a known method.

Examples of method for quantifying the iron concentration in a solution include phenanthroline absorption spectrophotometry, frame atomic absorption spectrometry, electrically heated atomic absorption spectrometry, and ICP emission spectrometry, after appropriately performing required treatments such as reduction.

In the case of adding ammonium iron citrate, when determining the theoretical value of iron concentration by calculation, if the value of iron content or the like cannot be obtained from the source, and the molecular formula of ammonium iron citrate is unknown or undetermined, 17.5% by mass (for reddish-brown color) or 15% by mass (for green color) can be used as the iron content of ammonium iron citrate for convenience.

The "medium additive" in the present disclosure refers to any substance added to a medium or a composition containing the substance. The medium additive may be one that replenishes a component that is already present in the medium, or one that adds a new component that is not present in the medium. The medium additive is typically provided in a liquid or powder form (including a granular form). The purpose of addition is not particularly limited.

The singular forms "a," "an," and "the" as used in the present disclosure may include a plurality of subjects unless specifically indicated otherwise.

The "and/or" as used in the present disclosure includes both the relationship represented by "and" and the relationship represented by "or".

The "comprising" as used in the present disclosure includes "consisting mainly of", "consisting substantially of" and "consisting of". The "consisting mainly of" includes "consisting substantially of" and "consisting of", and the "consisting substantially of" includes "consisting of".

For each numerical range in the present disclosure, the upper and lower limits indicated by "-" and "to" shall be included in the numerical range, respectively. For example, a description of "A-B" or "A to B" using numerical values A and B means that it is A or more and B or less. In addition, the descriptions of "A-B", "A to B" or "A or more and B or less" in numerical ranges described with stages in the present disclosure independently include both "A or more is preferred" and "B or less is preferred", and the lower or upper limit value may be replaced with an upper or lower limit value in other numerical ranges. The lower or upper limit value of a numerical range described in the present disclosure may also be replaced with a numerical value shown in Examples within the numerical range.

### (Embodiments)

Hereinafter, the present invention will be described in more detail using embodiments for carrying out the invention, but the present invention is not limited thereto.

These embodiments may be constituted singly or in combination. For definitions and details of each embodiment, reference can be made to the above "Description of terms".

Known techniques and procedures used in the present disclosure are well understood by those skilled in the art and can be performed in accordance with conventional methods.

In certain embodiments, a method for improving recombinant protein production in perfusion culture of an animal cell having a capacity to produce a recombinant protein, the method comprising adding cystine to a cell culture medium, is provided. In this method, the productivity of recombinant protein production is improved by improving recombinant protein production in perfusion culture.

As the indicators for recombinant protein production, those such as, but not limited to, recombinant protein production rate (amount of production per unit time) per culture tank, recombinant protein production rate (amount of production per unit time) per unit culture volume (medium volume), and recombinant protein production rate (amount of production per unit time) per unit cell can be used. Examples of these include, respectively, recombinant protein production per day, recombinant protein production per day per L, and recombinant protein production per day per cell. In certain embodiments, protein production is improved in at least one of the above indicators.

The amount of protein can be quantified by a known quantification method according to each protein. Examples of the protein quantification method include a method of quantifying the protein from the absorption coefficient (based on values calculated from amino acid sequences, values of reference proteins such as BSA, or the like) and absorption peak area by combining chromatography such as HPLC with absorption (e.g., absorption at 280 nm) of the protein; and a method of quantifying from absorption of purified/crude-purified proteins by chromatography (e.g., HPLC). Purified/crude-purified protein can also be quantified by BCA method, Bradford or Lowry method, or the like.

In certain embodiments, a method for improving recombinant protein production per cell in perfusion culture of an animal cell having a capacity to produce a recombinant protein, the method comprising adding cystine to a cell culture medium, is provided. As an indicator for recombinant protein production per cell, the recombinant protein production rate per cell (e.g., protein production per cell per day) can be used. When the recombinant protein is an antibody, the antibody production rate (SPR) is preferably used. In this method, the cost of recombinant protein production is suppressed and the productivity is improved by improving recombinant protein production per cell in perfusion culture.

The recombinant protein production per cell can be calculated by quantifying proteins by a quantification method according to each protein, and measuring the number of cells. The protein quantification method can be any method, and the number of cells can be measured manually, measured with a machine, measured by quantifying by biological and chemical methods using reagents capable of quantifying the number of cells, or measured by combining these.

The improvement of recombinant protein production per cell increases the ratio (purity) of recombinant proteins relative to cell-derived impurities and inclusions, making subsequent purification steps more convenient and suppressing costs. In addition, the improvement of protein quality at the time of production can also lead to the improvement of the quality of the final product, and the improvement of the quality of the final product is preferred from the viewpoint of safety when it becomes a pharmaceutical product, and the like.

Perfusion culture, in particular, may consume larger amounts of medium than other culture methods due to the reflux of the cell culture medium, thereby may lead to increase costs, which has been a point to be improved. The improvement of recombinant protein production per cell can result in larger production amounts of recombinant proteins when the same amount of cell culture medium is used (less medium is required to produce the same amount of recombinant proteins), which may lead to further cost reduction and productivity improvement.

In certain embodiments, a method for subjecting an animal cell having a capacity to produce a recombinant protein to perfusion culture, the method comprising: subjecting the animal cell having a capacity to produce a recombinant protein to perfusion culture in a cell culture primary medium; and adding cystine to the cell culture medium, is provided. In this method, a cell having a high capacity to produce a recombinant protein can be cultured.

In this method, a perfusion medium for perfusion culture different from the primary medium can also be used, and cystine may be added simultaneously or separately to the perfusion medium.

In certain embodiments, a method for producing a recombinant protein, the method comprising: subjecting the animal cell having a capacity to produce a recombinant protein to perfusion culture in a cell culture primary medium; adding cystine to the cell culture medium; and recovering a recombinant protein, is provided. In this method, recombinant proteins can be produced with excellent productivity.

In this method, a perfusion medium for perfusion culture different from the primary medium can also be used, and cystine may be added simultaneously or separately to the perfusion medium.

The perfusion culture in embodiments of the present disclosure is not limited to a specific perfusion culture method, and any perfusion culture method known at the time of implementation can be used. The cell separation means, medium supply means, culture conditions (CO₂ concentration, DO, culture temperature, culture time, or the like), culture scale, and culture tank are also not particularly limited, and can be appropriately selected from known conditions or the like and performed, considering the nature of the cells to be cultured, the nature of the recombinant protein to be produced, and the like. Examples of the culture conditions include culture temperature of 30-40°C, CO₂ concentration of 0-40%, DO of 20-70%, culture time of 1-90 days, culture fluid volume of 10 mL-2000 L, and/or perfusion volume of 0.1-30 culture volume per day. The animal cell is preferably cultured under conditions in which the recombinant protein is expressed or conditions suitable for expression of the recombinant protein.

The cell culture medium used for perfusion culture is not limited to specific ones, and can be appropriately selected considering the cells to be cultured and the recombinant proteins to be produced. The cell culture medium is preferably a chemically defined cell culture medium (CDM). The primary medium and the perfusion medium may have the same or similar compositions or different compositions. In embodiments of the present disclosure, when referred to "medium", "culture medium" or "cell culture medium" without specific notation, this means both the "primary medium", which is the medium at the start of perfusion culture, and the "perfusion medium", which is a new medium added for perfusion.

In embodiments of the present disclosure, typical examples of the animal cell having a capacity to produce a recombinant protein includes an animal cell containing a nucleic acid encoding a recombinant protein. The nucleic acid encoding the recombinant protein may be incorporated into the genome.

Although any animal cell can be used as the animal cell in embodiments of the present disclosure, an established cell line, particularly an animal cell line useful for the production of recombinant protein, is preferred.

The animal cell is preferably, but not limited to, an insect cell or mammalian cell, and more preferably a mammalian cell.

Examples of the suitable insect cell line can include a SF9 cell, a SF21 cell, and a SF+ cell.

Examples of the suitable mammalian cell line can include a BHK21 cell and a Chinese hamster ovary cell (CHO cell), and particularly preferably, a CHO cell. The CHO cell includes various sublines derived from CHO cell (e.g., a CHO-K1 cell, a CHO-S cell, a CHO-DXB11 cell (also referred to as CHO-DUKX cell or DuxB 11 cell) or a CHO-DG44 cell).

In embodiments of the present disclosure, the recombinant protein of interest is not limited to specific ones and any recombinant protein can be a target protein. A preferred example of the recombinant protein is an antibody.

Cystine in embodiments of the present disclosure can be any cystine as long as it functions as cystine (L-cystine) in the medium after adding. From the viewpoint of increasing the solubility of cystine, it is preferable that a cystine-containing solution having a pH adjusted to 10 or higher is added to the medium.

The timing and medium of adding cystine are not particularly limited, and cystine may be added to the primary medium before starting culturing and/or added to the perfusion medium and/or the primary medium after starting culturing. In addition, cystine may be added to the medium at one time or in multiple portions. Cystine is preferably added to the medium after starting culturing and even more preferably added to the perfusion medium. Especially when the cystine concentration is high, the addition of cystine after starting culturing can lead to better cell growth. When cystine is added after starting culturing, it may be added, for example, 1 hour or later after starting culturing, preferably 6 hours or later after starting culturing, more preferably 24 hours or later after starting culturing, and even more preferably 72 hours or later after starting culturing. Upon addition, cystine may be added directly to the medium, or a solution, suspension, or medium in which cystine has been dissolved or suspended may be added to the medium. When adding cystine to the perfusion medium, a cystine-containing medium may be prepared in advance and used as the perfusion medium.

The amount of cystine to be added is not particularly limited within the scope in which the effects of the invention are exerted, and example include an amount in which the concentration of cystine in the medium after adding (immediately after adding) is 0.5 mM to 10 mM. The concentration of cystine in the medium after adding (immediately after adding) is preferably 0.7 mM to 10 mM or 0.8 mM to 10 mM, more preferably 1 mM to 10 mM, even more preferably 1.5 mM to 10 mM, particularly preferably 2 mM to 10 mM, particularly more preferably 2.1 mM to 10 mM, or alternatively 2.5 mM to 10 mM. When cystine is added in these ranges, it can exert a particularly excellent effect. An embodiment in which cystine of a lower concentration than in the above ranges is added and then cystine is additionally added to fall within the above ranges is also included in the above embodiments.

Cystine in embodiments of the present disclosure is used as a cysteine source in animal cells. Thus, according to certain embodiments of the present disclosure, the cysteine source concentration in the culture medium can be suppressed.

That is, in a further embodiment of the present disclosure, the cysteine source concentration of the primary medium and/or the perfusion medium is, for example, 0.1 mM to 3 mM, preferably 0.1 mM to 2.1 mM, more preferably 0.1 mM to 1.5 mM. Preferably, the cysteine source concentration of the primary medium is within the above ranges. More preferably, the cysteine source concentration of the primary medium and the perfusion medium is in the above ranges. Cystine may be added in any of the aforementioned forms as a further addition to the cysteine source of these cysteine source concentrations.

In another embodiment, the cysteine source is a cysteine source other than cystine, and the cysteine source other than cystine is within the above concentration ranges. At this time, cystine may be added in addition to the cysteine source other than cystine of the above concentration ranges. The cysteine source other than cystine is preferably cysteine.

In another embodiment, the primary medium and/or the perfusion medium may not contain a cysteine source other than cystine and contain only cystine as the cysteine source.

Since cysteine can adversely affect medium stability, low cysteine source concentrations in the primary medium are advantageous in terms of medium stability.

In particular, perfusion culture requires a large amount of medium to be prepared in advance because perfusion culture uses extremely large amount of medium compared to other culture methods (for example, Fed-batch culture), and the high stability of the medium is a very advantageous property upon the preparation of large amount of medium. In other words, this effect can be said to be a particularly desirable effect in perfusion culture.

The "improving recombinant protein production" or "recombinant protein production is improved" in embodiments of the present disclosure means that recombinant protein production is improved as compared to when production is performed under conditions other than those of the present disclosure, but preferably recombinant protein production is improved as compared to when cysteine of an equimolar concentration to the cystine in terms of cysteine is added.

That is, in a further embodiment of the present disclosure, the recombinant protein production or the capacity to produce a recombinant protein is improved as compared to when cysteine of an equimolar concentration to the cystine in terms of cysteine is added. As shown in Examples, in the methods of embodiments of the present disclosure, the recombinant protein production or the capacity to produce a recombinant protein is surprisingly improved as compared to when cysteine of an equimolar concentration to the cystine in terms of cysteine is added.

In cystine, two cysteines are linked by an S-S bond, and the equimolar concentration in terms of cysteine means that the cystine concentration is half the molar concentration of cysteine.

In a further embodiment of the present disclosure, the recombinant protein production or the capacity to produce a recombinant protein is improved by 10% or more. The recombinant protein production or the capacity to produce a recombinant protein is preferably improved by 20% or more, more preferably by 30% or more, and even more preferably by 40% or more.

The capacity to produce a recombinant protein that can be achieved by further embodiments of the present disclosure includes, for example, 5-20 g/L/day, preferably 5.5-20 g/L/day, more preferably 6-20 g/L/day, and even more preferably 7-20 g/L/day. Another indicator for the capacity to produce a recombinant protein includes, for example, 20-200 pg/cell/day, preferably 24-150 pg/cell/day, and even more preferably 25-120 pg/cell/day. As other ranges, it may also include 20-40 pg/cell/day, 23 or 24-40 pg/cell/day, or 25-40 pg/cell/day.

The production of recombinant protein according to embodiments of the present disclosure may include, in addition to the aforementioned steps, a further variety of additional steps, and these are also included in embodiments of the present disclosure. Examples of the additional steps include purifying the recombinant protein or chemically or biologically modifying the recombinant protein.

Recombinant proteins are usually purified by a plurality of means in combination, and chromatography is preferably used. In particular, when the recombinant protein is an antibody, affinity chromatography, protein A chromatography, and the like are preferably used.

The chemical or biological modifications of the recombinant proteins include, but are not limited to, disulfide bond formation, glycosylation, lipidation, acetylation, phosphorylation, amidation, derivatization by a protecting group or the like, cleavage, or binding to a labeling component or a drug (e.g., a toxin). As an example, a method for producing an antibody conjugated to a drug such as toxin is also included in the method for producing a recombinant protein of the present disclosure, and this case may further include, as an additional step, conjugating an antibody to a drug. In this case, the antibody conjugated to the drug is included in the recombinant protein of the present disclosure.

In further embodiments of the present disclosure, embodiments pertaining to ammonium iron citrate described below are combined with the above embodiments.

That is, in further embodiments of the present disclosure, the cell culture medium contains ammonium iron citrate. Details of embodiments using ammonium iron citrate can be referred to the description mentioned below, and various embodiments mentioned below can be combined.

Another embodiment of the present disclosure is an agent for improving recombinant protein production in perfusion culture of an animal cell having a capacity to produce a recombinant protein, the agent comprising cystine as an active ingredient. This agent can improve the productivity of recombinant protein production by improving recombinant protein production in perfusion culture.

Embodiments of the agent are not limited to specific forms as long as the agent comprises cystine as the active ingredient, and the agent may consist solely of cystine, or may be in a form of a composition containing a carrier, a pH adjusting agent, a medium, or the like. The agent may also be in a liquid form or may be in a solid form such as a powder form (including granule form).

Embodiments of the agent are intended for use in improving recombinant protein production in perfusion culture of an animal cell having a capacity to produce a recombinant protein. The use is preferably described in a brochure for the sale, an instruction, a website about the product, or the like of the agent. In this case, the use may be described as an effect of the agent. Specific examples of the use of the agent include use as a medium additive before culture (when preparing a medium) or during culture.

For details of the embodiments and further embodiments of the agent described above, reference can be made to the details of the embodiments and respective embodiments of the method described above, and the configurations and details thereof are also incorporated into the present embodiments.

Embodiments related to the embodiments of the agent include the use of cystine as an agent for improving recombinant protein production in perfusion culture of an animal cell having a capacity to produce a recombinant protein, or the use of cystine for improving recombinant protein production in perfusion culture of an animal cell having a capacity to produce a recombinant protein. These embodiments can be performed in the same manner as the embodiments of the agent described above.

Although embodiments using cystine are described above, the present inventors have also found that ammonium iron citrate as an iron source also contributes to improve the productivity of recombinant proteins.

That is, a certain embodiment of the present disclosure is a method for improving recombinant protein production in perfusion culture of an animal cell having a capacity to produce a recombinant protein, the method comprising subjecting the animal cell having a capacity to produce a recombinant protein to perfusion culture in a cell culture medium containing ammonium iron citrate. In this method, the recombinant protein production in perfusion culture is improved, and the productivity of recombinant protein production is improved.

A certain embodiment of the present disclosure is a method for improving recombinant protein production per cell in perfusion culture of an animal cell having a capacity to produce a recombinant protein, the method comprising subjecting the animal cell having a capacity to produce a recombinant protein to perfusion culture in a cell culture medium containing ammonium iron citrate. In this method, the cost of recombinant protein production is suppressed and the productivity is improved by improving recombinant protein production per cell in perfusion culture.

The "improving recombinant protein production" or "recombinant protein production is improved" in embodiments of the present disclosure means that recombinant protein production is improved as compared to when production is performed under conditions other than those of the present disclosure, but preferably recombinant protein production is improved as compared to when the cell culture medium contains an alternative iron source to the ammonium iron citrate.

That is, in a further embodiment of the present disclosure, the recombinant protein production or the capacity to produce a recombinant protein is improved as compared to when the cell culture medium contains an alternative iron source to the ammonium iron citrate. As shown in Examples, in the methods of embodiments of the present disclosure, the recombinant protein production or the capacity to produce a recombinant protein is surprisingly improved as compared to when the cell culture medium contains an alternative iron source to the ammonium iron citrate.

In a further embodiment of the present disclosure, the alternative iron source is sodium ferrous citrate. The present embodiment has an excellent effect as compared to when sodium ferrous citrate, which is a common iron source, is used.

In a further embodiment of the present disclosure, the recombinant protein production or the capacity to produce a recombinant protein is improved by 10% or more. The recombinant protein production or the capacity to produce a recombinant protein is preferably improved by 20% or more, more preferably by 30% or more, and even more preferably by 40% or more.

The capacity to produce a recombinant protein that can be achieved by further embodiments of the present disclosure includes, for example, 5-20 g/L/day, preferably 5.5-20 g/L/day, more preferably 6-20 g/L/day, and even more preferably 7-20 g/L/day. Another indicator for the capacity to produce a recombinant protein includes, for example, 20-200 pg/cell/day, preferably 24-150 pg/cell/day, and even more preferably 25-120 pg/cell/day. As other ranges, it may also include 20-40 pg/cell/day, 23 or 24-40 pg/cell/day, or 25-40 pg/cell/day.

In certain embodiments, a method for subjecting an animal cell having a capacity to produce a recombinant protein to perfusion culture, the method comprising: subjecting the animal cell having a capacity to produce a recombinant protein to perfusion culture in a cell culture medium containing ammonium iron citrate, is provided. In this method, a cell having a high capacity to produce a recombinant protein can be cultured.

A certain embodiment of the present disclosure is a method for reducing a cell growth rate in perfusion culture of an animal cell having a capacity to produce a recombinant protein, the method comprising culturing the animal cell having a capacity to produce a recombinant protein in a cell culture medium containing ammonium iron citrate. In this method, the productivity in recombinant protein production is improved by reducing the cell growth rate in perfusion culture.

In certain embodiments of the present disclosure, cell growth can be suppressed while the recombinant protein production is improved, and in this case the required amount of media or the like can be suppressed while the produced amount of the desired recombinant protein is maintained. It can also reduce cell-derived impurities per the same amount of recombinant protein, contributing to reduction of the purification cost and improvement of the quality.

The "reducing a cell growth rate" or "cell growth rate is reduced" in embodiments of the present disclosure means that the cell growth rate is reduced as compared to when production is performed under conditions other than those of the present disclosure, but preferably the cell growth rate is reduced as compared to when the cell culture medium contains an alternative iron source to the ammonium iron citrate.

In a further embodiment of the present disclosure, the alternative iron source is sodium ferrous citrate. The present embodiment has an excellent effect as compared to when sodium ferrous citrate, which is a common iron source, is used.

In a further embodiment of the present disclosure, the cell growth rate is reduced by 10% or more. The cell growth rate is preferably reduced by 20% or more, more preferably by 30% or more, and even more preferably by 40% or more.

A certain embodiment of the present disclosure is a method for reducing an amount of culture fluid discharged in perfusion culture of an animal cell having a capacity to produce a recombinant protein, the method comprising: subjecting the animal cell having a capacity to produce a recombinant protein to perfusion culture in a cell culture medium containing ammonium iron citrate, wherein the amount of culture fluid discharged is an amount of culture fluid discharged in a breeding step, and the breeding step is a step of discharging the culture fluid from a culture tank and adding a fresh medium in an equal amount to the discharged culture fluid to the culture tank. In this method, the productivity of recombinant protein production is improved by reducing the amount of culture fluid discharged. The reduction of the amount of culture fluid discharged is brought about by the suppression of cell growth.

The "reducing an amount of culture fluid discharged" or "amount of culture fluid discharged is reduced" in embodiments of the present disclosure means that the amount of culture fluid discharged in perfusion culture of an animal cell having a capacity to produce a recombinant protein is reduced as compared to when production is performed under conditions other than those of the present disclosure. The indicator for the amount of culture fluid discharged include, but is not limited to, the total amount of culture fluid discharged in the whole breeding step or in a certain period of time therein and the amount of culture fluid discharged in a unit time (discharging rate of culture fluid).

In certain embodiments of the present disclosure, the amount of culture fluid discharged can be reduced while the recombinant protein production is improved, and in this case the required amount of media or the like can be suppressed while the produced amount of the desired recombinant protein is maintained. In addition, for recombinant proteins secreted extracellularly, the reduction in amount of culture fluid discharged enables the production at a higher concentration, which is advantageous in terms of the quality thereafter and the purification cost.

In a further embodiment of the present disclosure, the amount of culture fluid discharged in perfusion culture of an animal cell having a capacity to produce a recombinant protein is reduced as compared to when the cell culture medium contains an alternative iron source to the ammonium iron citrate. In the present embodiment, as shown in Examples, the amount of culture fluid discharged is surprisingly reduced as compared to when the cell culture medium contains an alternative iron source to the ammonium iron citrate.

In a further embodiment of the present disclosure, the alternative iron source is sodium ferrous citrate. The present embodiment has an excellent effect as compared to when sodium ferrous citrate, which is a common iron source, is used.

In a further embodiment of the present disclosure, the amount of culture fluid discharged is reduced by 10% or more. The amount of culture fluid discharged is preferably reduced by 20% or more, more preferably by 30% or more, and even more preferably by 40% or more.

Ammonium iron citrate in embodiments of the present disclosure may also be denoted as ammonium iron(III) citrate or ammonium ferric citrate. Ammonium iron citrate is added to the primary medium and/or the perfusion medium. Upon addition, ammonium iron citrate may be added directly to the medium, or a solution, suspension, or medium in which ammonium iron citrate has been dissolved or suspended may be added to the medium.

The amount of ammonium iron citrate to be added is not particularly limited within the scope in which the effects of the invention is exerted, and example include an amount in which the iron concentration (iron ion concentration) derived from ammonium iron citrate in the medium after adding is 10 mg/L to 300 mg/L. In the present disclosure, such iron concentrations may be described using expressions, for example, "an amount that provides an iron concentration of 10 mg/L to 300 mg/L." The iron concentration is preferably 20 mg/L to 200 mg/L or 30 mg/L to 200 mg/L, more preferably 40 mg/L to 200 mg/L or 40 mg/L to 150 mg/L, even more preferably 50 mg/L to 200 mg/L or 50 mg/L to 150 mg/L. When ammonium iron citrate is in these ranges, it can exert a particularly excellent effect.

In another embodiment, the amount of ammonium iron citrate to be added is not particularly limited within the scope in which the effects of the invention is exerted, and for example, the cell culture medium contains 50 mg/L to 1500 mg/L ammonium iron citrate. The concentration of ammonium iron citrate is preferably 100 mg/L to 1000 mg/L or 150 mg/L to 1000 mg/L, more preferably 200 mg/L to 1000 mg/L or 200 mg/L to 750 mg/L, even more preferably 250 mg/L to 1000 mg/L or 250 mg/L to 750 mg/L. When ammonium iron citrate is in these ranges, it can exert a particularly excellent effect.

In certain embodiments, the cell culture medium may contain an additional iron source to the ammonium iron citrate described above.

When the additional iron source is contained, the amount thereof is not particularly limited, but examples of the amount of iron source include amounts in which the iron concentration in the medium, including ammonium iron citrate, is from 1 mg/L to 400 mg/L, from 10 mg/L to 300 mg/L, from 20 mg/L to 200 mg/L, from 30 mg/L to 200 mg/L, from 40 mg/L to 200 mg/L, from 40 mg/L to 150 mg/L, from 50 mg/L to 200 mg/L, or from 50 mg/L to 150 mg/L.

For details of the embodiments and further embodiments pertaining to ammonium iron citrate described above, reference can be made to the details of the embodiments and specific embodiments pertaining to cystine described above, and the configurations and details thereof are also incorporated into the embodiments concerning ammonium iron citrate.

For example, for the recombinant protein, animal cell having a capacity to produce a recombinant protein, perfusion culture, or increase in recombinant protein production in embodiments pertaining to ammonium iron citrate, reference can be made to the descriptions of the recombinant protein, animal cell having a capacity to produce a recombinant protein, perfusion culture, or increase in recombinant protein production in embodiments pertaining to cystine described above.

Another embodiment of the present disclosure is an agent for improving recombinant protein production in perfusion culture of an animal cell having a capacity to produce a recombinant protein, the agent comprising ammonium iron citrate as an active ingredient. This agent can improve the productivity of recombinant protein production by improving recombinant protein production in perfusion culture.

Another embodiment of the present disclosure is an agent for reducing a cell growth rate in perfusion culture of an animal cell having a capacity to produce a recombinant protein or an agent for reducing an amount of culture fluid discharged in perfusion culture of an animal cell having a capacity to produce a recombinant protein, the agent comprising ammonium iron citrate as an active ingredient.

Embodiments of the agent are not limited to specific forms as long as the agent comprises ammonium iron citrate as the active ingredient, and the agent may consist solely of ammonium iron citrate, or may be in a form of a composition containing a carrier, a pH adjusting agent, a medium, or the like. The agent may also be in a liquid form or may be in a solid form such as a powder form (including granule form).

Embodiments of the above agents are each intended for use in improving recombinant protein production in perfusion culture of an animal cell having a capacity to produce a recombinant protein, for use in reducing a cell growth rate in perfusion culture of an animal cell having a capacity to produce a recombinant protein, or for use in reducing an amount of culture fluid discharged in perfusion culture of an animal cell having a capacity to produce a recombinant protein. The use is preferably described in a brochure for the sale, an instruction, a website about the product, or the like of the agent. In this case, the use may be described as an effect of the agent. Specific examples of the use of the agent include use as a medium additive before culture (when preparing a medium) or during culture.

For details of the embodiments and further embodiments described above, reference can be made to the details of the embodiments and respective embodiments of the method described above (including the parts into which the embodiments pertaining to cystine are incorporated), and the configurations and details thereof are also incorporated into the present embodiments.

Embodiments related to the embodiments of the agent include the use of ammonium iron citrate as an agent for improving recombinant protein production in perfusion culture of an animal cell having a capacity to produce a recombinant protein, the use of ammonium iron citrate for improving recombinant protein production in perfusion culture of an animal cell having a capacity to produce a recombinant protein, the use of ammonium iron citrate as an agent for reducing a cell growth rate in perfusion culture of an animal cell having a capacity to produce a recombinant protein, the use of ammonium iron citrate for reducing a cell growth rate in perfusion culture of an animal cell having a capacity to produce a recombinant protein, the use of ammonium iron citrate as an agent for reducing an amount of culture fluid discharged in perfusion culture of an animal cell having a capacity to produce a recombinant protein, or the use of ammonium iron citrate for reducing an amount of culture fluid discharged in perfusion culture of an animal cell having a capacity to produce a recombinant protein. These embodiments can also be performed in the same manner as the embodiments of the agent described above.

Another embodiment of the present disclosure is a cell culture medium for perfusion culture of an animal cell having a capacity to produce a recombinant protein, the cell culture medium comprising cystine and ammonium iron citrate. This medium can improve the productivity of recombinant protein production by exerting the actions and effects of both the embodiments pertaining to cystine and the embodiments pertaining to ammonium iron citrate described above. The medium may be used as a primary medium and may be used as a perfusion medium. In addition, the medium includes both a liquid medium and a powder medium.

For details of the embodiments and further embodiments described above, reference can be made to the details of the embodiments and respective embodiments of the method described above (including the parts into which the embodiments pertaining to cystine are incorporated), and the configurations and details thereof are also incorporated into the embodiments described above.

It should be noted that respective methods and the like described by the above embodiments or the like are not intended to limit the present invention, and disclosed to show examples. The technical scope of the present invention is defined by the descriptions of the claims, and those skilled in the art can make various design changes within the technical scope of the invention described in the claims.

For example, in a certain embodiment, a recombinant protein produced by an embodiment of the present disclosure is provided. Another embodiment relates to a pharmaceutical composition or medicament containing the recombinant protein, use of the recombinant protein in manufacture of a medicament, or a method for treating a disease, comprising administering the recombinant protein. Another embodiment relates to a method for producing a pharmaceutical composition or pharmaceutical formulation, comprising any of the steps according to embodiments of the present disclosure or any of the methods according to embodiments of the present disclosure, thereby obtaining a recombinant protein, and further mixing the recombinant protein with a carrier.

### [Examples]

Hereinafter, the present invention will be described with reference to Examples, but these Examples do not limit the present invention. The commercially available reagents, equipment, and the like mentioned in Examples were used in accordance with the manufacturer's instructions or usual methods, unless otherwise indicated.

### [Materials and Methods]

### (Cells)

For all experiments, CHO cell lines producing humanized IgG antibodies (DXB-11 line; G. Urlaub et al, Proc. Natl. Acad. Sci. USA 77:4216-4220, 1980) were used.

### (Cell Culture Conditions)

### Perfusion culture

A chemically defined cell culture medium was added to a bioreactor of 2L capacity, into which the above CHO cell lines were seeded, and culturing was started at the culture fluid volume of 1 L and 37°C. DO was controlled to 50%, and pH was controlled to 7.15 ± 0.05. From day 1 of culturing, perfusion was performed with a chemically defined perfusion medium at 1.0 to 5.0 culture volume per day using an alternating tangential flow perfusion filtration system (Refine Technologies, LLC, Hanover, NJ) with a 2.0 µm hollow fiber filter (Repligen Corporation, MA, US).

### Repeat culture

A sufficient amount of the above CHO cell line culture fluid was centrifuged to remove the supernatant, and then the culture was suspended in a chemically defined perfusion medium to be 100 x 10⁵ cells/mL, and culturing was started in a 125 mL triangular flask (Corning Incorporated, NY, US) at the culture fluid volume of 20 mL and 37°C. Every day until day 4 of the culturing, the culture fluid was centrifuged to remove the supernatant, and then the remainder was resuspended in a chemically defined fresh perfusion medium to be 100 x 10⁵ cells/mL, and the culturing was continued at the culture fluid volume of 20 mL and 37°C.

### (Analytical method)

The viable cell density and viability were measured using Vi-CELL (Beckman Coulter, Inc., CA, US), and the titer was measured with the centrifugal supernatant of the culture fluid by the Protein A-HPLC method (calculated based on the absorbance coefficient calculated from the amino acid sequence and the peak area of A280).

### [Example 1]

### Cysteine Source Enhancement Evaluation with Cysteine and Cystine

This Example demonstrated that the enhancement with cystine as a cysteine source improved the productivity greater than the enhancement with cysteine. Evaluation in perfusion culture (cystine was added from 72 hours after starting culturing) was performed using condition (1) (perfusion medium containing 3.379 mM cysteine), condition (2) (perfusion medium containing 5.333 mM cysteine), and condition (3) (perfusion medium containing 1.942 mM cysteine and 1.696 mM cystine). A mixture of 1.25-fold concentrated CELLiST(R) Basal media (Ajinomoto Co., Inc.: BASAL10) and CELLiST(R) Feed media (Ajinomoto Co., Inc.: FEED2) at 94 : 6 were used as the cell culture primary medium and the perfusion medium. The results are shown in Figure 1. The productivity was improved in both conditions (2) and (3), in which the cysteine source was enhanced, compared with condition (1). However, when compared condition (2) with condition (3), in which the enhancement was performed with equivalent molar concentration in terms of cysteine, condition (3), in which the enhancement was performed as cystine, surprisingly improved the productivity greater.

### [Example 2]

### Iron Source Enhancement Evaluation with Sodium Ferrous Citrate and ammonium iron citrate

This Example demonstrated that the enhancement with ammonium iron citrate as an iron source greatly improved the productivity while suppressed the cell growth. The cell culture primary medium and perfusion medium were prepared without an iron source, and the iron final concentration was adjusted by adding corresponding amounts of sodium ferrous citrate concentrate and ammonium iron citrate concentrate each. CELLiST(R) Basal media (Ajinomoto Co., Inc.: BASAL10) was used as the cell culture primary medium and the perfusion medium. Evaluation was performed in repeat culture. The results are shown in Figure 2. Enhancing the iron source improved the productivity, but surprisingly, enhancing with ammonium iron citrate improved the productivity greater while slowed the cell growth.

It should be noted that the contents of disclosures of patents, patent applications, and publications cited in the present disclosure are incorporated into the present disclosure in their entirety by reference.

## Claims

1. A method for improving recombinant protein production in perfusion culture of an animal cell having a capacity to produce a recombinant protein, the method comprising:
adding cystine to a cell culture medium.

2. A method for improving recombinant protein production per cell in perfusion culture of an animal cell having a capacity to produce a recombinant protein, the method comprising:
adding cystine to a cell culture medium.

3. The method according to claim 1 or 2, wherein the recombinant protein production is improved as compared to when cysteine is added at an equimolar concentration to the cystine in terms of cysteine.

4. The method according to claim 3, wherein the recombinant protein production is improved by 10% or more.

5. The method according to claim 1 or 2, the method comprising adding cystine one hour or later after starting culturing.

6. The method according to claim 1 or 2, the method comprising adding cystine 6 hours or later after starting culturing.

7. The method according to claim 1 or 2, the method comprising adding cystine 24 hours or later after starting culturing.

8. The method according to claim 1 or 2, the method comprising adding cystine 72 hours or later after starting culturing.

9. The method according to claim 1 or 2, wherein a cysteine source concentration in a primary medium in the perfusion culture is 0.1 mM to 2.1 mM.

10. A method for subjecting an animal cell having a capacity to produce a recombinant protein to perfusion culture, the method comprising:
subjecting the animal cell having a capacity to produce a recombinant protein to perfusion culture in a cell culture primary medium having a cysteine source concentration of 0.1 mM to 2.1 mM; and
adding cystine to a cell culture medium 72 hours or later after starting culturing.

11. The method according to claim 10, wherein the capacity to produce a recombinant protein is improved as compared to when cysteine of an equimolar concentration to the cystine in terms of cysteine is added.

12. The method according to claim 11, wherein the capacity to produce a recombinant protein is improved by 10% or more.

13. A method for producing a recombinant protein, the method comprising:
subjecting an animal cell having a capacity to produce a recombinant protein to perfusion culture in a cell culture primary medium having a cysteine source concentration of 0.1 mM to 2.1 mM;
adding cystine to a cell culture medium 72 hours or later after starting culturing; and
recovering a recombinant protein.

14. The method according to claim 13, wherein the recombinant protein production is improved as compared to when cysteine of an equimolar concentration to the cystine in terms of cysteine is added.

15. The method according to claim 14, wherein the recombinant protein production is improved by 10% or more.

16. The method according to any one of claims 1, 2, and 10 to 15, wherein a concentration of cystine after adding is 0.5 mM to 10 mM.

17. The method according to any one of claims 1, 2, and 10 to 15, wherein a concentration of cystine after adding is 1 mM to 10 mM.

18. The method according to any one of claims 1, 2 and 10 to 15, wherein a concentration of cystine after adding is 1.5 mM to 10 mM.

19. The method according to any one of claims 1, 2, and 10 to 15, wherein a concentration of cystine after adding is 2 mM to 10 mM.

20. The method according to any one of claims 1, 2, and 10 to 15, the method comprising adding a cystine-containing solution adjusted to pH 10 or higher.

21. The method according to any one of claims 1, 2, and 10 to 15, wherein the recombinant protein is an antibody.

22. The method according to any one of claims 1, 2, and 10 to 15, wherein the animal cell having a capacity to produce a recombinant protein contains a nucleic acid encoding a recombinant protein.

23. The method according to any one of claims 1, 2, and 10 to 15, wherein the animal cell is a mammalian cell.

24. The method according to claim 23, wherein the mammalian cell is a CHO cell.

25. The method according to claim 24, wherein the CHO cell is a CHO-K1 cell, a CHO-S cell, a CHO-DXB 11 cell, or a CHO-DG44 cell.

26. The method according to any one of claims 1, 2, and 10 to 15, wherein the cell culture medium in the perfusion culture contains ammonium iron citrate.

27. An agent for improving recombinant protein production in perfusion culture of an animal cell having a capacity to produce a recombinant protein, the agent comprising cystine as an active ingredient.

28. A method for improving recombinant protein production in perfusion culture of an animal cell having a capacity to produce a recombinant protein, the method comprising:
subjecting the animal cell having a capacity to produce a recombinant protein to perfusion culture in a cell culture medium containing ammonium iron citrate.

29. A method for improving recombinant protein production per cell in perfusion culture of an animal cell having a capacity to produce a recombinant protein, the method comprising:
subjecting the animal cell having a capacity to produce a recombinant protein to perfusion culture in a cell culture medium containing ammonium iron citrate.

30. The method according to claim 28 or 29, wherein the recombinant protein production is improved as compared to when the cell culture medium contains an alternative iron source to the ammonium iron citrate.

31. The method according to claim 30, wherein the alternative iron source is sodium ferrous citrate.

32. The method according to claim 30, wherein the recombinant protein production is improved by 10% or more.

33. A method for subjecting an animal cell having a capacity to produce a recombinant protein to perfusion culture, the method comprising:
subjecting the animal cell having a capacity to produce a recombinant protein to perfusion culture in a cell culture medium containing ammonium iron citrate.

34. A method for reducing a cell growth rate in perfusion culture of an animal cell having a capacity to produce a recombinant protein, the method comprising:
culturing the animal cell having a capacity to produce a recombinant protein in a cell culture medium containing ammonium iron citrate.

35. The method according to claim 33 or 34, wherein the cell growth rate is reduced as compared to when the cell culture medium contains an alternative iron source to the ammonium iron citrate.

36. The method according to claim 35, wherein the alternative iron source is sodium ferrous citrate.

37. The method according to claim 35, wherein the cell growth rate is reduced by 10% or more.

38. A method for reducing an amount of culture fluid discharged in perfusion culture of an animal cell having a capacity to produce a recombinant protein, the method comprising:
subjecting the animal cell having a capacity to produce a recombinant protein to perfusion culture in a cell culture medium containing ammonium iron citrate,
wherein the amount of culture fluid discharged is an amount of culture fluid discharged in a breeding step, and the breeding step is a step of discharging the culture fluid from a culture tank and adding a fresh medium in an equal amount to the discharged culture fluid to the culture tank.

39. The method according to claim 38, wherein the amount of culture fluid discharged is reduced as compared to when the cell culture medium contains an alternative iron source to the ammonium iron citrate.

40. The method according to claim 39, wherein the alternative iron source is sodium ferrous citrate.

41. The method according to claim 39, wherein the amount of culture fluid discharged is reduced by 10% or more.

42. A method for producing a recombinant protein, the method comprising:
subjecting an animal cell having a capacity to produce a recombinant protein to perfusion culture in a cell culture medium containing ammonium iron citrate; and
recovering a recombinant protein.

43. The method according to any one of claims 28, 29, 33, 34, and 38 to 42, wherein the cell culture medium contains an amount of ammonium iron citrate that provides an iron concentration of 30 mg/L to 200 mg/L.

44. The method according to any one of claims 28, 29, 33, 34, and 38 to 42, wherein the cell culture medium contains an amount of ammonium iron citrate that provides an iron concentration of 40 mg/L to 200 mg/L.

45. The method according to any one of claims 28, 29, 33, 34, and 38 to 42, wherein the cell culture medium contains an amount of ammonium iron citrate that provides an iron concentration of 50 mg/L to 150 mg/L.

46. The method according to any one of claims 28, 29, 33, 34, and 38 to 42, wherein the recombinant protein is an antibody.

47. The method according to any one of claims 28, 29, 33, 34, and 38 to 42, wherein the animal cell having a capacity to produce a recombinant protein contains a nucleic acid encoding a recombinant protein.

48. The method according to any one of claims 28, 29, 33, 34, and 38 to 42, wherein the animal cell is a mammalian cell.

49. The method according to claim 48, wherein the mammalian cell is a CHO cell.

50. The method according to claim 49, wherein the CHO cell is a CHO-K1 cell, a CHO-S cell, a CHO-DXB 11 cell, or a CHO-DG44 cell.

51. The method according to any one of claims 28, 29, 33, 34, and 38 to 42, the method comprising adding cystine to the cell culture medium.

52. An agent for improving recombinant protein production in perfusion culture of an animal cell having a capacity to produce a recombinant protein, the agent comprising ammonium iron citrate as an active ingredient.

53. A cell culture medium for perfusion culture of an animal cell having a capacity to produce a recombinant protein, the cell culture medium comprising cystine and ammonium iron citrate.
